# EUROPÄISCHE PATENTANMELDUNG

(11) **EP 1 922 987 A1**
(43) Veröffentlichungstag der Anmeldung: **21.05.2008**
(21) Anmeldenummer: 06124342.4
(22) Anmeldetag: 17.11.2006
(51) Int. Cl.: A61B 5/00, B01D 61/24, G01N 1/34

(54) **Probenahmevorrichtung und Probenahmeverfahren**

(71) Anmelder: Trace Analytics GmbH, 38106 Braunschweig (DE)
(72) Erfinder: Künnecke, Wolfgang, 38116, Braunschweig (DE); Hartlep, Michael, 38102, Braunschweig (DE); Giesenberg, Jens, 38304, Wolfenbüttel (DE); Lehmann, Matthias, 38106, Braunschweig (DE)
(74) Vertreter: Eisenführ, Speiser & Partner

(57) **Zusammenfassung**

Probenahmevorrichtung zum Gewinnen einer Probe eines Analyten, umfassend eine Zuleitung (30) und eine Ableitung (40) sowie eine Analytaufgabekammer (13) in fluidleitender Verbindung zwischen der Zuleitung und der Ableitung, wobei die Analytaufgabekammer eine Öffnung (14) besitzt, die mit einer analytdurchlässigen Membran (15) zum Durchtretenlassen des Analyten aus einem Bereich außerhalb der Analytaufgabekammer in die Analytaufgabekammer versehen ist, dadurch gekennzeichnet, dass die Fläche der Öffnung der Analytaufgabekammer höchstens das 400fache der Mindest-Querschnittsfläche der Ableitung, besonders bevorzugt höchstens das 100fache und besonders bevorzugt das 50-80fache der Mindest-Querschnittsfläche der Ableitung beträgt.
Mit dieser Vorrichtung soll die Totzeit zwischen den Durchtreten des Analyten durch die Membran und dem Nachweis am Sensor verringert werden.

## Beschreibung

Die vorliegende Erfindung betrifft eine Probenahmevorrichtung zum Gewinnen einer Probe eines Analyten, sowie Verfahren zum Gewinnen einer Probe eines Analyten.

In der Verfahrenstechnik und Medizin, insbesondere bei der Beobachtung eines Bioreaktors und der Langzeitbestimmung des Gehalts vorgewählter Substanzen im menschlichen Körper, ist es häufig notwendig, das Vorhandensein und gegebenenfalls die Konzentration eines vorgewählten Analyten in einem Medium schnell und möglichst kontinuierlich zu bestimmen. Aus hygienischen und/oder medizinischen Gründen ist es dabei häufig nicht möglich, unmittelbar Material des zu untersuchenden Mediums beispielsweise in einer Biopsie wiederholt oder fortgesetzt zu entnehmen. Üblicherweise wird deshalb eine Probe des Analyten aus dem Medium durch ein Dialyseverfahren gewonnen. Dabei wird eine mit einer Dialyse- oder Gasdiffusionsmembran als "Messfenster" versehene Sonde in das zu untersuchende Medium eingeführt und gegebenenfalls für längere Zeit implantiert. Die Sonde wird mit einem Transportmedium kontinuierlich oder pulsweise gespült. Durch die Membran tritt der Analyt in das Transportmedium einer in Analyt-Strömungsrichtung hinter der Membran liegenden Analytaufgabekammer ein und wird durch die Sonde durch einen Sondenausgang aus dem Bereich des zu untersuchenden Mediums, insbesondere einem Bioreaktor oder einem menschlichen oder tierischen Körper, heraustransportiert. Das gegebenenfalls analytbeladene Transportmedium kann dann durch einen oder mehrere Sensoren analysiert werden. Beispiele solcher Sonden und zugehörige Probenahmeverfahren sind beschrieben in der DE 44 26 694, US 3640269, US 4694832, US 6632315 B2, US 6811542 B2, WO 99/45982 A2 und WO 01/06928 A1.

Ein Problem bisheriger Sonden und entsprechenden Probenahmeverfahren war die begrenzte Transportgeschwindigkeit des Analyten in den Sonden und dementsprechend die lange Totzeit zwischen dem ersten Durchtreten des Analyten durch die Membran und dem ersten Nachweis des Analyten am Sondenausgang. Beispielsweise benötigt der in der WO 99/45982 A2 beschriebene Herzkatheter eine Totzeit von 15 bis 20 Minuten, bevor ein messbares Signal zur Verfügung steht, dazu unten mehr.

Zum Lösen dieses Problems wurde einerseits versucht, die Membranfläche zu vergrößern. Hierzu wurden beispielsweise Mikrodialysesonden eingesetzt, die Hohlfasern mit einem Einzelfaser-Durchmesser von etwa 500 µm umfassen. Nachteilig daran ist, dass sich die Membran beim Herausziehen der Sonde insbesondere aus Gewebe ablösen und im Körper verbleiben kann. Zum Erreichen einer hohen Transportgeschwindigkeit werden Mikrodialysesonden häufig mit einem hohen Innendruck des Transportmediums beaufschlagt. Der hohe Innendruck belastet zusätzlich die Verbindungen zwischen den Hohlfasern und der übrigen Sonde, so dass es häufig zu Rissen in der Hohlfaser oder zum Ausreißen der Hohlfaser aus der Sonde kommt.

Zudem bewirken die bei üblichem Betrieb eines Bioreaktors oder bei einer Sondenimplantation in einem menschlichen oder tierischen Patientenkörper allfällig auftretenden Druckschwankungen starke Schwankungen der Dialyserate. Dieser Effekt wird noch dadurch verstärkt, dass die verwendeten Membranmaterialien in wässriger Umgebung stark quellen und dadurch sehr flexibel werden. So entstehen nach Implantation oder Eintauchen in eine Messlösung bei der Verwendung von Hohlfasern sehr pulsationsfähige "Schlauchbeutel" als Messfenster. Die Dialyserate schwankt dann sehr stark, weil sich das Membranzellvolumen im Inneren bei wechselndem Gegendruck ständig verändern kann. Selbst kleinste Bewegungen oder Druckschwankungen sind deutlich erkennbar. Abhilfe kann dazu nur der Betrieb bei hohem Innendruck schaffen, was die Gefahr des Platzens wiederum erhöht. Dementsprechend sind solche Mikrodialysesonden üblicherweise ungeeignet für ein pulsweises Beaufschlagen der Sonde mit dem Transportmedium.

Auch aus fluiddynamischer Sicht weisen die sich bei der Verwendung von Hohlfasern ergebenden zylindrischen Dialysekammern Nachteile auf. In der Analytaufgabekammer ergeben sich konstruktionsbedingt unterschiedlich durchströmte Bereiche, Ecken und Richtungsumkehrungen, in denen sich Luftblasen festsetzen können. Da Mikrodialysesonden üblicherweise Analytaufgabekammern mit kleinen Innendurchmessern aufweisen, lassen sich darin nur geringe Fließgeschwindigkeiten aufbauen, so dass das Herausspülen der Luftblasen sehr schwierig ist. Die Luftblasen verändern das mit Transportmedium gefüllte Volumen der Analytaufgabekammer und/oder die wirksame Membranfläche und bewirken so erhebliche Messfehler.

Und schließlich ist wegen des erwünschten hohen Stofftransportrate des Analyten in die Analytaufgabekammer der maximale Volumenstrom des Transportmediums in einer Mikrodialysesonde begrenzt. Übliche Mikrodialysesonden können nur mit Transportmedium-Volumenströmen von 0,3-0,5 µl/min betrieben werden. Für den Transport des Transportmediums aus der Analytaufgabekammer zum Sondenausgang wird so bei einer angenommenen Transportstrecke von 30 cm und einem üblichen Sonden-Innendurchmesser von 75 µm eine Zeit (Totzeit) von 160 s, bei einem Sonden-Innendurchmesser von 150 µm sogar von 630 s benötigt. Mit Verringerung des Sonden-Innendurchmessers erhöht sich der Druck des Transportmediums bei gleicher Transportgeschwindigkeit um den Faktor 4, so dass ein Mindest-Sonden-Innendurchmesser zum Vermeiden des Reißens oder Platzens der Membran eingehalten werden muss.

Ebenfalls bekannt sind Probenahmesonden, bei denen das Transportmedium nicht kontinuierlich, sondern pulsweise ausgetauscht wird. Dabei diffundiert der Analyt aus dem zu untersuchenden Medium wiederum durch eine Membran (Dialyse- oder Gasdiffusionsmembran) in eine in Diffusionsrichtung hinter der Membran liegende Analytaufgabekammer in das Transportmedium. Je nach Zeitdauer zwischen zwei aufeinanderfolgenden Transportmedium-Austauschpulsen wird der Analyt bei gleicher Analyt-Anfangskonzentration des zu untersuchenden Mediums in der Analytaufgabekammer unterschiedlich stark angereichert. Beispielsweise beschreibt die US 6852500 eine Mikrodialysesonde und einen Glucose-Durchflusssensor, wobei die Mikrodialysesonde pulsweise durch Transportmedium durchströmt wird. Unter Berücksichtigung des Innenvolumens der Mikrodialysesonde ergibt sich so eine Dauer von 9 Minuten, bis das gesamte Volumen der Analytaufgabekammer den Sondenausgang passiert und den Glucose-Durchflusssensor erreicht hat. Die so ermittelten Messergebnisse können daher nur Durchschnittswerte sein und geben den tatsächlichen Verlauf der Glucosekonzentration nur zeitverzögert und gedämpft wieder.

Aufgabe der vorliegenden Erfindung war es daher, eine Probenahmevorrichtung anzugeben, mit denen die oben beschriebenen Nachteile herkömmlicher Probenahmevorrichtungen vermieden oder verringert werden können. Die Probenahmevorrichtung sollte insbesondere eine geringe Totzeit zwischen dem Eintreten eines Analyten in eine Analytaufgabekammer und dem Passieren des Probenahmevorrichtungs-Ausgangs ermöglichen. Vorzugsweise sollte eine Strecke von 30 cm Länge zwischen der Analytaufgabekammer und dem Probenahmevorrichtungs-Ausgang innerhalb von 2 Minuten durch ein Transportmedium durchströmt werden können, ohne dass dabei Drücke auftreten, die den sicheren Betrieb der Probenahmevorrichtung gefährden und insbesondere ein Reißen der Probenahmevorrichtung auslösen können. Ferner sollte ein entsprechendes Probenahmeverfahren angegeben werden.

Die Aufgabe wird gelöst durch eine Probenahmevorrichtung gemäß Anspruch 1. Vorteilhafte Weiterbildungen der Erfindung sind in den Unteransprüchen beschrieben. Ein erfindungsgemäßes Probenahmeverfahren ist in Anspruch 17 beschrieben.

Erfindungsgemäß umfasst eine Probenahmevorrichtung zum Gewinnen einer Probe eines Analyten eine Zuleitung und eine Ableitung sowie eine Analytaufgabekammer in fluidleitender Verbindung zwischen Zuleitung und Ableitung. Die Analytaufgabekammer besitzt eine Öffnung, die mit einer analytdurchlässigen Membran zum Durchtretenlassen des Analyten aus einem zu untersuchenden Medium aus einem Bereich außerhalb der Analytaufgabekammer ("außen") in die Analytaufgabekammer versehen ist. Die erfindungsgemäße Probenahmevorrichtung zeichnet sich dadurch aus, dass die Fläche der Öffnung der Analytaufgabekammer höchstens das 400fache, bevorzugt höchstens das 200fache und besonders bevorzugt das 40-80fache der Mindest-Querschnittfläche der Ableitung beträgt. Dabei ist unter der Mindest-Querschnittfläche der Ableitung die Fläche des Querschnitts zu verstehen, der für die Ableitung und die Analytaufgabekammer innerhalb der Probenahmevorrichtung die Kleinste ist. Üblicherweise wird die Mindest-Querschnittfläche durchflussmengenbegrenzend wirken. Mit ,,Fläche der Öffnung" ist diejenige Fläche gemeint, die den Durchtritt des Analyten von außen in die Analytaufgabekammer am stärksten begrenzt. Üblicherweise wird die Fläche die Fläche der fensterartigen Durchtritts-Öffnung der Probenahmevorrichtung sein. Die Fläche kann jedoch auch anstelle eines einzigen Fensters aus einer Vielzahl von Fenstern bestehen, die zusammenwirkend insgesamt eine Öffnung der Analytaufgabekammer in Richtung auf das zu untersuchende Medium ergeben. Die Fläche der Öffnung ist dann die Gesamtfläche der einzelnen Fenster. Die Form der Öffnung (als einzelnes Fenster oder als Vielzahl an Fenstern) kann beliebig gewählt werden. Der Fachmann wird dabei insbesondere die Auswirkungen der Öffnungsgeometrie auf die Stabilität der erfindungsgemäßen Probenahmevorrichtung berücksichtigen.

Überraschenderweise hat sich gezeigt, dass mit der erfindungsgemäß vorgesehenen, im Vergleich zu herkömmlichen Probenahmevorrichtungen kleinen Öffnung am Ende der Ableitung der Probenahmevorrichtung noch immer ein messbares Analyt-Signal erhalten werden kann. Dies war überraschend, da zu erwarten gewesen war, dass eine kleine Öffnung nur eine langsame Diffusion des Analyten in die Analytaufgabekammer gestatten würde, die Konzentration des Analyen in der Analytaufgabekammer dementsprechend pro Zeiteinheit niedrig und wegen der durch Turbulenz verursachten Verdünnung beim Transport des Analyten von der Analytaufgabekammer durch die Ableitung am Ende der Ableitung ein kaum noch messbares Signal erhalten würde. Tatsächlich hat sich jedoch herausgestellt, dass zwar eine Verdünnung beim Transport des Analyten durch die Ableitung stattfindet, da der Analyt jedoch ursprünglich nur in einem kleinen Volumen in Diffusionsrichtung hinter der kleinen Öffnung angereichert war, fällt diese Verdünnung nur gering aus. Ferner ermöglicht die im Vergleich zur Mindest-Querschnittfläche kleine Öffnung der Analytaufgabekammer, die Analytaufgabekammer mit einem hohen Volumenstrom des Transportmediums zu beschicken, ohne dass ein Zerreißen der Membran befürchtet werden muss. Durch den hohen Volumenstrom kann das Festsetzen von Blasen in Analytdiffusionsrichtung hinter der Membran in der Analytaufgabekammer vermieden werden; dies erhöht die Messsicherheit zusätzlich und auf einfache Weise.

In bevorzugten Ausführungsformen der erfindungsgemäßen Probenahmevorrichtung beträgt die Länge der Ableitung, gemessen vom Rand der Öffnung der Analytaufgabekammer bis zum Ende der Ableitung
a) zumindest 20 cm, vorzugsweise 30 cm bis 80 cm und besonders bevorzugt 35 cm bis 50 cm, oder
b) 3 bis 15 cm, vorzugsweise 5 bis 10 cm und besonders bevorzugt 6 bis 8 cm beträgt.

Besonders geeignete sind die in a) beschriebenen Abmessungen für zentralvenöse Katheter und vergleichbare Probenahmesonden, während die in b) beschriebenen Abmessungen besonders geeignet für Venenverweilkatheter sind.

Bei diesen Längen der Ableitung und damit Analyt-Transportstrecken bleibt die durch Turbulenz hervorgerufene Verdünnung des Analyten in einem Rahmen, der eine Konzentrationsbestimmung üblicher Analyten wie Glucose aus einem implantierten Katheter bei einem Menschen oder Tier ermöglicht.

Die Mindest-Querschnittsfläche der Ableitung einer erfindungsgemäßen Probenahmevorrichtung beträgt in bevorzugten Ausführungsformen
a) 0,03 bis 3 mm², vorzugsweise 0,1 bis 0,8 mm² und besonders bevorzugt 0,2 bis 0,5 mm², oder
b) 0,008 bis 0,8 mm², vorzugsweise 0,018 bis 0,5 mm² und besonders bevorzugt 0,03 bis 0,2 mm² beträgt.

Wiederum sind die in a) beschriebenen Mindest-Querschnittsflächen besonders geeignet für zentralvenöse Katheter, während die in b) beschriebenen Mindest-Querschnittsflächen besonders geeignet für Venenverweilkatheter aufgrund der insgesamt kleineren Baugröße sind.

Förderlich ist es ferner, das Volumen der Analytaufgabekammer in Abhängigkeit von der Fläche der Öffnung der Analytaufgabekammer zu wählen. Vorzugsweise beträgt das Verhältnis zwischen Öffnungsfläche und Analytaufgabekammervolumen insbesondere bei zentralvenösen Kathetern 15 mm²/15 mm³ bis 90 mm²/15 mm³, stärker bevorzugt 20 mm²/15 mm³ bis 45 mm²/15 mm³ und besonders bevorzugt 30 mm²/15 m³. In herkömmlichen Mikrodialysevorrichtungen ist das Verhältnis deutlich größer und beträgt häufig etwa 16 mm²/2 mm³. Dies führt zu einem langgezogenen "Pfropfen" des Analyten im Transportmediums, der beim Transport noch weiter auseinandergezogen wird und somit für eine vollständige Vermessung erheblich mehr Zeit in Anspruch nimmt als mit einer erfindungsgemäßen Probenahmevorrichtung.

Die Membran einer erfindungsgemäßen Probenahmevorrichtung besitzt in bevorzugten Ausführungsformen der Erfindung eine Ausschlussgrenze von 80 kDa, vorzugsweise von 60 kDa und besonders bevorzugt von 20 kDa. Die Membran einer erfindungsgemäßen Probenahmevorrichtung ist dementsprechend passierbar für kleine Analyten, wie sie insbesondere bei der Untersuchung eines menschlichen oder tierischen Patienten üblicherweise von Interesse sind. Interessierende Analyten und entsprechende abgeleitete Größen, die mit einer erfindungsgemäßen Probenahmevorrichtung nachgewiesen werden können, sind vorzugsweise Glucose, Lactat, pH, pO₂ und pCO₂, Methanol, Ethanol, Formiat, Acetat, Glutamin, Glutamat, Harnstoff, Harnsäure, Phosphat, Antikörper, Wachstumsfaktoren und Hormone. Das Membranmaterial ist dementsprechend bevorzugt ausgewählt aus der Gruppe bestehend aus Cellulose und deren Derivate, Celluloseacetat, PTFE, Polycarbonat, Polypropylen, Polyamide, Polysulfone, wobei zum Nachweisen von Glucose insbesondere eine Celluloseacetat--Membran bevorzugt ist.

Die erfindungsgemäße Probenahmevorrichtung umfasst vorzugsweise ferner eine Leitung für einen weiteren, vom Transportmedium verschiedenen Medienstrom. Die weitere Leitung mündet in bevorzugten Ausführungsformen im Gebrauch in das zu untersuchende Medium. Auf diese Weise kann gleichzeitig mit einer Beprobung eines die Probenahmevorrichtung umgebenden Mediums in dieses Medium ein Medienstrom eingeleitet oder entnommen werden. Dabei umfasst eine besonders bevorzugte Ausführungsform eine Öffnung, die die Analytaufgabekammer mit der Leitung des weiteren Medienstroms verbindet, wobei optional keine Öffnung zu dem außerhalb der Probenahmevorrichtung befindlichen Medium vorgesehen ist. Eine solche Probenahmevorrichtung kann beispielsweise verwendet werden zum Einleiten einer Kalibrierlösung über die Öffnung zum Kalibrieren der Probenahmevorrichtung, wobei zur Untersuchung des außerhalb der Probenahmevorrichtung befindlichen Mediums dieses dann in die Leitung eingesaugt wird. Das Einleiten einer Kalibrier- oder anderen Infusionslösung und das Einsaugen des zu untersuchenden Mediums kann durch eine Pumpe durchgeführt werden, vorzugsweise in regelmäßigen Abständen. Dadurch ist die Exposition der Öffnung gegenüber dem zu untersuchenden Medium steuerbar.

In einer weiteren Ausführungsform umfasst die erfindungsgemäße Probenahmevorrichtung zwei Öffnungen, von denen eine im bestimmungsgemäßen Gebrauch mit dem zu untersuchenden Medium und eine mit der weiteren Leitung in Verbindung steht. Auf diese Weise kann beispielsweise der Analyt in der Analytaufgabekammer verdünnt werden.

Die weitere Leitung kann jedoch auch von dem zu untersuchenden Medium getrennt sein. Dies ermöglicht eine genauere Kalibrierung durch die Analytaufgabekammer.

Die weitere Leitung kann auch mit zusätzlichen Sensoren, z.B. Faseroptiken zur Messung von Sauerstoff oder pH-Wert, versehen werden. Dazu werden Lichtleiter eingesetzt, deren Spitze beispielsweise mit einem Indikator farbstoff beschichtet ist und die sich in Abhängigkeit von der Konzentration der umgebenden Analyten z.B. in ihrer Fluoreszenz verändert.

Die erfindungsgemäße Probenahmevorrichtung ist vorzugsweise ein Probenahmekatheter, vorzugsweise für eine Blutuntersuchung in Form einer Braunüle, und ferner bevorzugt ein Zentralvenen-Katheter zur Anwendung an einem Säugetier, insbesondere einem Menschen. Ebenfalls bevorzugt sind erfindungsgemäße Probenahmevorrichtungen in Form einer Bioreaktorsonde..

Die Zuleitung und Ableitung in der erfindungsgemäßen Probenahmevorrichtung sind vorzugsweise nebeneinanderliegend in einem einheitlichen Baukörper enthalten. Diese Anordnung ist besonders zweckmäßig in medizinischen Probenahmevorrichtungen, insbesondere in Form von Zentralvenen-Kathetern und Venenverweilkanülen.

Die erfindungsgemäße Probenahmevorrichtung ist vorzugsweise mit einem Detektor zum Nachweisen des Analyten verbunden. Ebenfalls bevorzugt ist es, die Probenahmevorrichtung mit einer Pumpe zum Pumpen eines Transportmediums durch die Zuleitung und/oder Ableitung der Probenahmevorrichtung zu verbinden. Die Ableitung der Probenahmevorrichtung steht zweckmäßigerweise, optional über die Pumpe, in fluidleitender Verbindung mit dem Detektor.

Soweit die Probenahmevorrichtung mit einer Pumpe versehen ist, besitzt die Pumpe in bevorzugten Ausführungsformen eine Steuerung zum Betätigen der Pumpe zu einem vorgewählten Zeitpunkt. Die Pumpe kann so, insbesondere zu vorgewählten Zeitpunkten, ein- und ausgeschaltet werden, so dass die Probenahmevorrichtung pulsweise mit dem Transportmedium durchströmt wird. Besonders bevorzugt ist die Steuerung so eingerichtet, dass die Pumpe 20 bis 90, vorzugsweise 30 Sekunden lang ausgeschaltet ist, so dass Analyt durch die Membran in die Analytaufgabekammer gelangen kann, und anschließend so lange eingeschaltet ist, bis das analytbeladene Trägermedium-Volumen aus der Analytaufgabekammer durch die Ableitung aus der Probenahmevorrichtung herausgepumpt und vorzugsweise in einen Detektor hineingepumpt ist. Bei einer Analytaufgabekammergröße von 10-20 mm³ ist es bevorzugt, die Pumpe 20 bis 90 Sekunden lang auszuschalten. Ferner ist es bevorzugt, zwischen der Pumpe und der Zu- und/oder Ableitung einen Strömungsunterbrecher mit einer Steuerung vorzusehen, so dass der Strom von Transportmedium durch Zu- und Ableitung unabhängig vom Betrieb der Pumpe ein- und ausgeschaltet werden kann. Der Strömungsunterbrecher funktioniert dann wie eine zu vorgewählten Zeitpunkten ein- und ausgeschaltete Pumpe, wobei die Pumpe jedoch nicht ausgeschaltet werden muss.

Besonders bevorzugt ist eine Probenahmevorrichtung, deren Zu- und Ableitung über einen Verteiler mit dem Detektor so verbunden sind, dass zum Spülen ein Transportmedium vom Verteiler ohne Passieren der Analytaufgabekammer zum Detektor geleitet werden kann, und zum Messen das Transportmedium vom Verteiler unter Passieren der Analytaufgabekammer zum Detektor geleitet werden kann.

Diese Ausführungsform der erfindungsgemäßen Probenahmevorrichtung nutzt auf besonders vorteilhafte Weise aus, dass in der Zeit, die für das Beladen des ruhenden Transportmediums in der Analytaufgabekammer mit Analyten benötigt wird, der Detektor gespült werden kann. Diese erfindungsgemäße Probenahmevorrichtung ermöglicht so, eine rasche Abfolge von Messungen vorzunehmen, ohne dass zum Spülen verwendetes Transportmedium dabei die Analytaufgabekammer passieren und gegebenenfalls mit minimalen Mengen des Analyten beladen werden müsste, was zu einer Fehlkalibrierung des Detektors führen könnte.

Die Erfindung wird nachfolgend anhand der Figuren und Beispiele näher beschrieben, ohne dass diese den Schutzbereich der Patentansprüche einschränken sollen:

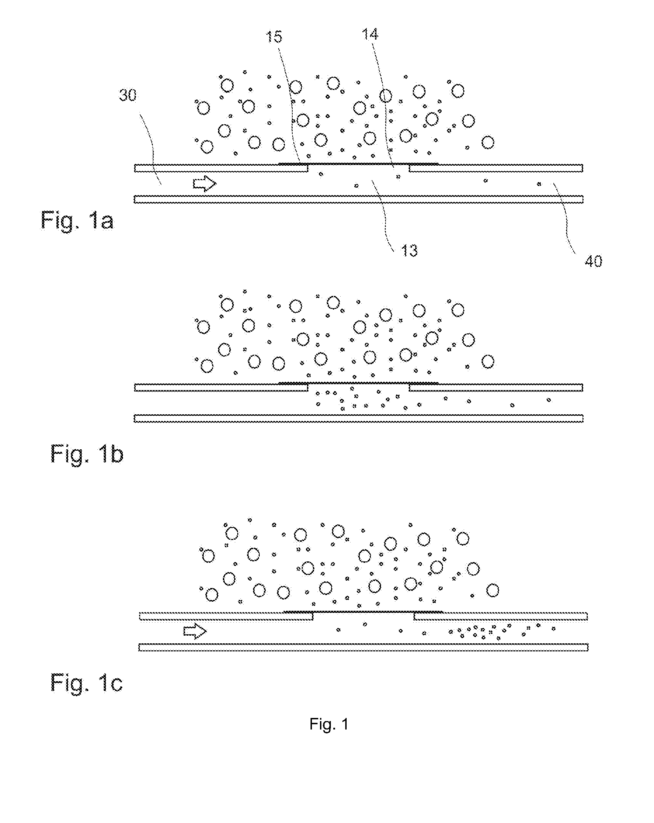

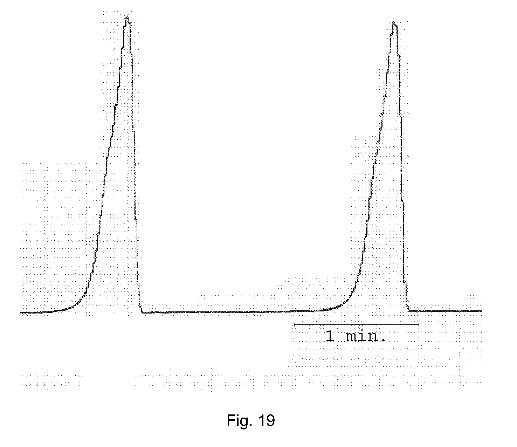

In Figur 1 wird der Prozess der Probenahme schematisch dargestellt. Im Ausgangszustand wird Transportlösung durch die Aufgabekammer 13 gefördert (Figur 1a). Dabei tritt kontinuierlich eine kleine Menge Analyt durch die Membran 15 in die Aufgabekammer und wird sofort zum Ablauf 40 weiter transportiert. Zum Anreichern wird die Transportlösung für eine bestimmte Zeit angehalten so dass sich eine größere Menge Analyt in der Aufgabekammer anreichern kann (Figur1b). Anschließend wird der Transportstrom wieder eingeschaltet und die angereicherte Menge an Analyt wird zum Ablauf weitertransportiert (Figur 1c). Dabei bildet sich eine Anhäufung von Analyt, die in einem nachgeschalteten Detektor ein peakförmiges Signal, wie in Figur 19 dargestellt, erzeugt. Die Höhe, Fläche oder Anfangssteigung kann dann zur Ermittlung der Konzentration des Analyten herangezogen werden.

### Beispiel 1: Zentralvenöse Katheter

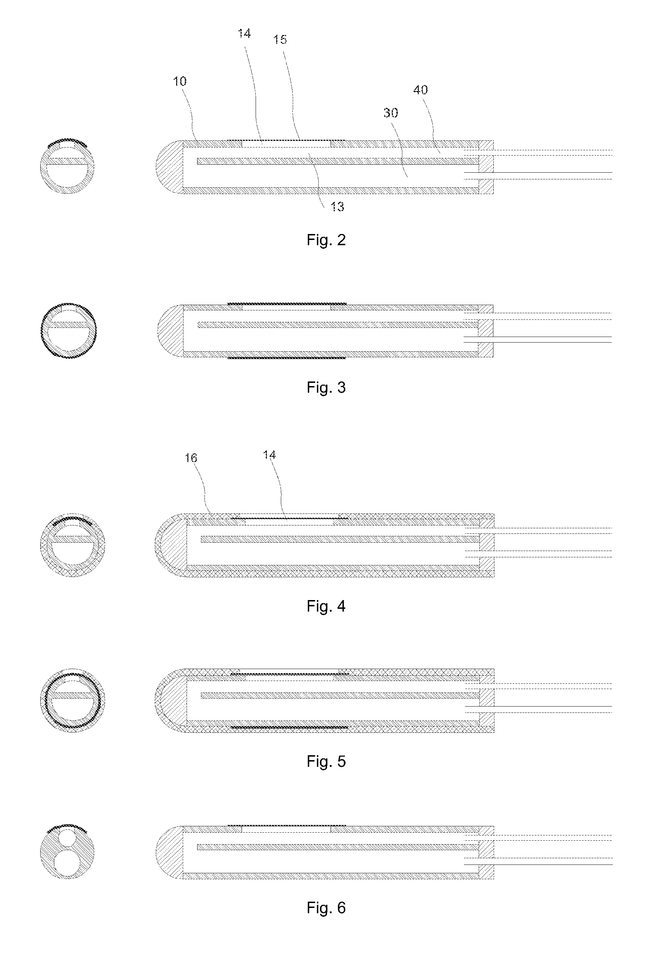

Zentralvenöse Katheter werden bisher routinemäßig zur Infusion eingesetzt. Erfindungsgemäße zentralvenöse Katheter sind in den Figuren 2 bis 12 gezeigt. Die Katheter können entweder nur zur Dialyse (Fig. 2 bis 11) oder als Mehrfunktionskatheter ausgelegt werden. Bei Mehrfunktionskathetern sind verschiedenste Katheterquerschnitte realisierbar, beispielsweise ein oder zwei Messkanäle mit zwei Infusionskanälen. Zusätzlich können weitere Kanäle für Führungsdrähte oder zum Einführen von z.B. faseroptischen Sensoren vorgesehen werden.

Die erfindungsgemäßen Katheter 10 besitzen eine Zuleitung 30 und eine Ableitung 40. Sie sind im wesentlichen röhrenförmig ausgebildet. An einer Seite des Katheters 10 ist eine Öffnung 14 vorgesehen. Die Öffnung 14 ist mit einer Membran 15 bedeckt oder ausgefüllt. Der Raum 13 im Ablauf 40, der sich über die Länge der Öffnung in Haupterstreckungsrichtung des Katheters 10 erstreckt, bildet eine Analytaufgabekammer. Die Analytaufgabekammer ist im Betrieb mit einem Transportmedium für einen durch die Membran 15 in die Analytaufgabekammer diffundierenden Analyten gefüllt (nicht dargestellt).

Bei dem in Figur 2 gezeigten Aufbau wird ein Membranstück 15 außen auf die Katheterfläche aufgebracht, so dass die Öffnung 14 mit der Membran dicht verschlossen wird. Falls unterschiedliche Lumenquerschnitte der Ableitung 40 verwendet werden, soll der kleinere Querschnitt zum Signalabtransport (Ausgang) verwendet werden.

In der Ausführungsform gemäß Figur 3 wird eine Membran 15 als Schlauch von außen über den Katheter 10 gebracht und verklebt, verschweißt oder anderweitig fluiddicht fixiert, so dass der Analyt in die Analytaufgabekammer diffundieren, jedoch kein Transportmedium durch die Öffnung nach außen gelangen kann.

In einer weiteren Ausführungsform ist zusätzlich zu der aus Figur 3 bekannten Membran eine weitere Schicht 16 , in der das Fenster über der Öffnung 14 freigelassen wird, über den Katheter angebracht. Dadurch wird verhindert, dass die Membran an ihren Kanten beim Ein- oder Herausführen des Katheters in einen Patientenkörper beschädigt wird. Die weitere Schicht kann die gesamte Spitze des Katheters wie eine Socke umgeben (Fig.4 und 5) oder schlauchförmig die Katheterspitze freilassen (nicht dargestellt).

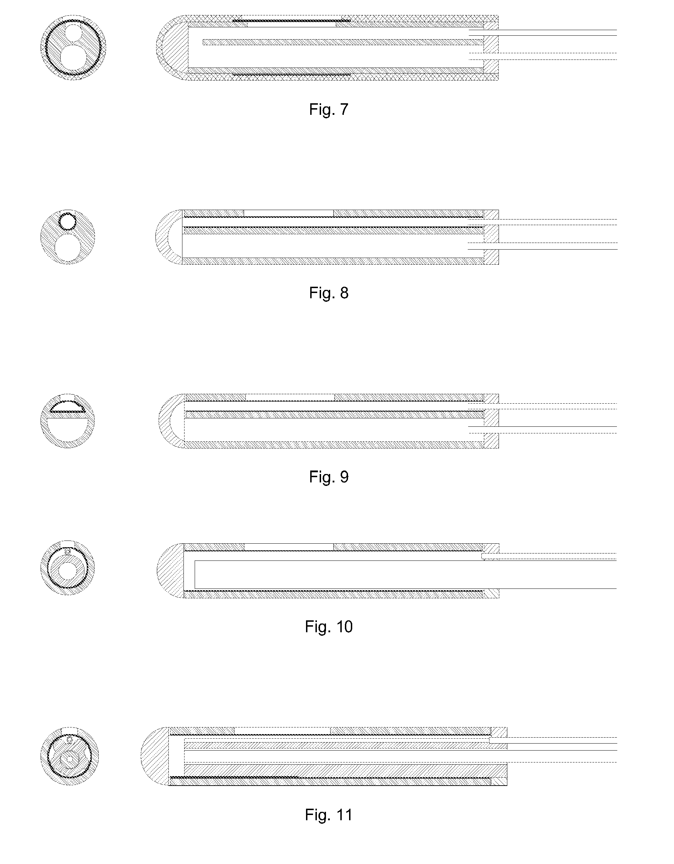

Das Innenlumen der Analytaufgabekammer kann beliebig gestaltet sein. Während die Probenahmevorrichtungen gemäß den Figuren 2 bis 5 einen D-förmigen Querschnitt der Analytaufgabekammer aufweisen, ist der Querschnitt in den Kathetern gemäß den Figuren 6 bis 8 rund. Dabei entspricht der Katheter gemäß Figur 6 ansonsten dem gemäß Figur 2 und der Katheter gemäß Figur 7 dem gemäß Figur 5.

In Figur 8 ist ein Katheter gezeigt, bei dem die Membran 15 nicht auf der Katheteraußenseite, sondern an der Innenseite der Analytaufgabekammer mit rundem Querschnitt angeordnet ist. Figur 9 zeigt einen entsprechenden Katheter mit D-förmigem Querschnitt. Figur 10 zeigt einen erfindungsgemäßen Katheter der in Figur 8 dargestellten Art, wobei der Querschnitt der Analytaufgabekammer kreisförmig ist.

Die Analytaufgabekammer des Katheters gemäß den Figuren 2 bis 9 war gebildet als Ausnehmung eines im wesentlichen zylindrischen Hohlkörpers, wobei die Zuleitung 30 und Ableitung 40 eine einstückig aus dem übrigen Katheter geformte gemeinsame Wand besaßen. Die Figuren 10 und 11 zeigen alternative erfindungsgemäße Katheter, bei denen der im wesentlichen zylindrische Katheter einen inneren Hohlraum umfasst, in den eine als Zuleitung 30 dienende Röhre eingeschoben ist. Die Röhre ist teilweise beabstandet von der Wandung des inneren Hohlraums des Katheters und bildet so einen freien Raum. Der freie Raum ist mit der Ableitung 40 verbunden. Wie in den vorherigen Figuren üblich ist der Katheter mit einem Fenster zum Eintretenlassen des Analyten versehen. Das Fenster ist auf der Wandung des inneren Hohlraums des Katheters mit einer Membran überdeckt, um ein Austreten des Transportmediums aus dem Fenster zu verhindern. Über die Röhre der Zuleitung 30 kann Transportmedium in den freien Raum im inneren Hohlraum des Katheters austreten; ein zu untersuchender Analyt kann durch das Fenster und die Membran in den inneren Hohlraum des Katheters eintreten und sich in der in Eintrittsrichtung hinter dem Fenster liegenden Analytaufgabekammer im Transportmedium anreichern. Das Transportmedium kann dann durch die Ableitung 40 abgeleitet und einem Sensor (nicht dargestellt) zugeführt werden.

Der Katheter gemäß Figur 11 unterscheidet sich von dem gemäß Figur 10 dadurch, dass die als Zuleitung 30 dienende Röhre auf ihrer Außenwand Abstandhalter aufweist.

Ferner kann zusätzlich oder alternativ zu der als Zuleitung 30 dienenden Röhre auch die Ableitung 40 mit einer Röhre versehen sein. Eine solche Ausführungsform ist in Figur 11 dargestellt.

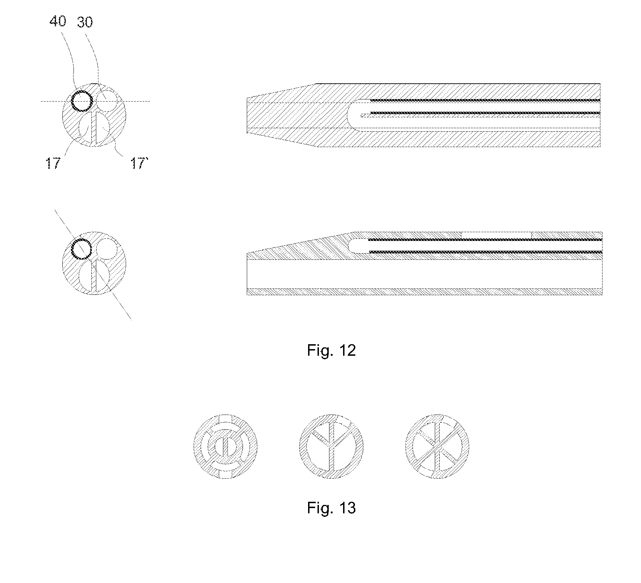

Eine erfindungsgemäße Probenahmevorrichtung kann außer den in den Figuren 1 bis 11 dargestellten Bestandteilen noch weitere Bestandteile umfassen. Beispielsweise zeigt Figur 12 einen erfindungsgemäßen Katheter, der neben einer Zuleitung 30 und einer Ableitung 40 noch eine zusätzliche röhrenförmige, bis zur Katheterspitze reichende Ausnehmung 17/17'umfasst. Die weitere Ausnehmung ist eingerichtet als Leitung für eine Infusion wie bei einem herkömmlichen Katheter üblich. Sie ist zweigeteilt ausgeführt und erlaubt so die getrennte Leitung zweier Fluidströme in bzw. aus einem zu untersuchenden menschlichen oder tierischen Patientenkörper.

Weitere alternative Querschnitte erfindungsgemäßer Probenahmevorrichtungen sind in Figur 13 dargestellt.

### Beispiel 2: Venenverweilkanülen

Venenverweilkanülen, auch als Braunülen bekannt, werden in der Infusiontherapie häufig verwendet. Sie werden in kleinen peripheren Venen, beispielsweise auf der Hand oder Fuß eingesetzt. Sie dienen üblicherweise zur Infusion von Lösungen und zur häufigen Blutentnahme. Braunülen sind dementsprechend zumeist kleiner, kürzer und dünner als zentralvenöse Katheter.

Zur Applikation einer Venenverweilkanüle wird zunächst mittels einer im Lumen der Kanüle angeordneten Nadel die Haut eines Patienten und das zu behandelnde Gefäß durchstochen. Dann wird die Nadel langsam herausgezogen und ein darüberliegendes flexibles Kunststoffteil in die Vene eingeführt.

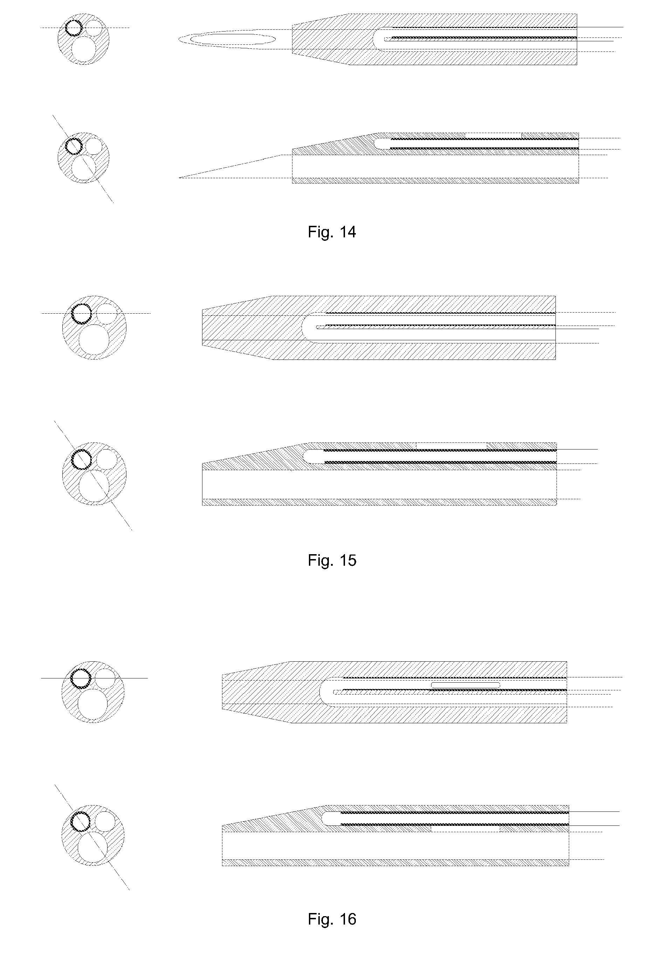

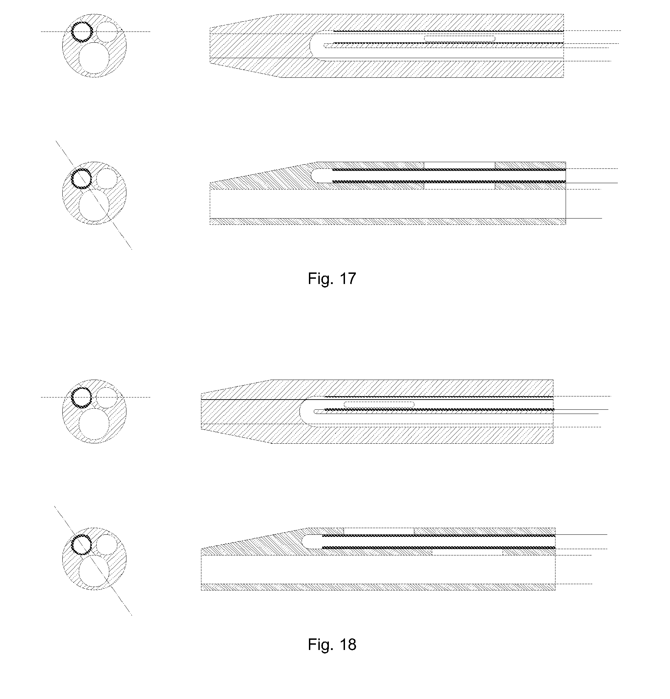

Die Figuren 14 bis 18 zeigen Ausführungsformen erfindungsgemäßer Venenverweilkatheter jeweils in der Aufsicht und der Seitenansicht. Die Katheter besitzen einen Aufbau der in Figur 12 gezeigten Art, wobei die zur Katheterspitze reichende Ausnehmung ausgebildet ist zur Aufnahme der zur Applikation verwendeten Nadel. Figur 14 zeigt einen entsprechenden Katheter mit Nadel, Figur 15 einen Katheter ohne Nadel. Figur 16 zeigt einen weiteren Katheter ohne Nadel, dabei ist die Öffnung 14 jedoch nicht an der Außenseite des Katheters angeordnet, sondern verbindet die zur Katheterspitze reichende Ausnehmung mit der Analytaufgabekammer. Eine solche Anordnung der Öffnung 14 gestattet es, ein in der inneren Ausnehmung befindliches Medium zu beproben. Die Figuren 17 und 18 zeigen eine weitere Ausführungsform der in Figur 16 gezeigten Art. Diese Ausführungsform umfasst zusätzlich eine weitere Öffnung 14 zur Verbindung der Analytaufgabekammer mit einem außerhalb des Katheters befindlichen Mediums. Die beiden Öffnungen können einander gegenüberliegend (Figur 17) oder gegeneinander versetzt (Figur 18) angeordnet sein.

### Beispiel 3: Anwendung einer erfindungsgemäßen Probenahmevorrichtung

Ein herkömmlicher Doppel-D-Katheter mit einem Aussendurchmesser von 1,6 mm wurde mit einer membranbedeckten Öffnung von 30 mm² (1,5 mm x 20mm rechteckig, Breite x Länge) versehen entsprechend Fig 2. Der Katheter wurde mit seiner Öffnung in eine wässrige Glucoselösung getaucht. Ein Transportmedium aus 100 mM Kaliumphosphat-Pufferlösung, pH 7,5, wurde 60 s lang mit 1,5 ml/min durch die Zu- und Ableitung gefördert. Anschließend wurde das Transportmedium 30 Sekunden lang nicht durch die Zu- und Ableitung befördert, so dass in dieser Zeit Analyt durch die Membran in die Analytaufgabekammer gelangen konnte. Am Auslass des Katheters wurde ein Durchfluss-Sensor mit einer immobilisierten Glukose-Oxidase-Elektrode angeordnet. Die Distanz zwischen der Öffnung und dem Durchfluss-Sensor betrug 100 cm. Alle Untersuchungen fanden bei Raumtemperatur statt. Das in den Durchfluss-Sensor beförderte, gegebenenfalls glucosehaltige Transportmedium wurde mittels eines amperometrischen Messverstärkers bei einem Potential von 350 mV vermessen. Die entstehenden Signale wiesen eine scharfe Peakform auf. Figur 19 zeigt die Signalabhängigkeit (Peakmaximum) in Bezug auf die Glukose-Konzentration der Glucoselösung. Die gesamte Durchlaufzeit pro Messsignal betrug weniger als 60 Sekunden. Die Kalibrationsgerade verläuft durch den Ursprung, so dass Einpunkt-Kalibrierungen, z.B. mittels externen Messgeräten möglich sind.

## Patentansprüche

1. Probenahmevorrichtung zum Gewinnen einer Probe eines Analyten, umfassend eine Zuleitung und eine Ableitung sowie eine Analytaufgabekammer in fluidleitender Verbindung zwischen der Zuleitung und der Ableitung, wobei die Analytaufgabekammer eine Öffnung besitzt, die mit einer analytdurchlässigen Membran zum Durchtretenlassen des Analyten aus einem Bereich außerhalb der Analytaufgabekammer in die Analytaufgabekammer versehen ist, **dadurch gekennzeichnet, dass** die Fläche der Öffnung der Analytaufgabekammer höchstens das 400fache der Mindest-Querschnittsfläche der Ableitung, besonders bevorzugt höchstens das 100fache und besonders bevorzugt das 50-80fache der Mindest-Querschnittsfläche der Ableitung beträgt.

2. Probenahmevorrichtung nach Anspruch 1, **dadurch gekennzeichnet, dass** die Länge der Ableitung, gemessen vom Rand der Öffnung der Ableitung an,
a) zumindest 20 cm, vorzugsweise zumindest 30 cm und besonders bevorzugt 35 bis 50 cm beträgt, oder
b) 3 bis 15 cm, vorzugsweise 5 bis 10 cm und besonders bevorzugt 6 bis 8 cm beträgt.

3. Probenahmevorrichtung nach einem der vorherigen Ansprüche, **dadurch gekennzeichnet, dass** die Mindest-Querschnittsfläche der Ableitung einer erfindungsgemäßen Probenahmevorrichtung
a) 0,03 bis 3 mm², vorzugsweise 0,1 bis 0,8 mm² und besonders bevorzugt 0,2 bis 0,5 mm² oder
b) 0,008 bis 0,8 mm², vorzugsweise 0,018 bis 0,5 mm² und besonders bevorzugt 0,03 bis 0,2 mm² beträgt.

4. Probenahmevorrichtung nach einem der vorherigen Ansprüche, **dadurch gekennzeichnet, dass** die Fläche der Öffnung der Analytaufgabekammer 0,05 bis 100 mm², vorzugsweise 0,1 bis 50 mm² und besonders bevorzugt 0,5 bis 30 mm² beträgt.

5. Probenahmevorrichtung nach einem der vorherigen Ansprüche, **dadurch gekennzeichnet, dass** das Verhältnis zwischen Öffnungsfläche und Analytaufgabekammervolumen 15 mm²/15 mm³ bis 90 mm²/15 mm³, stärker bevorzugt 20 mm²/15 mm³ bis 45 mm²/15 mm³ und besonders bevorzugt 30 mm²/15 m³ beträgt.

6. Probenahmevorrichtung nach einem der vorherigen Ansprüche, **dadurch gekennzeichnet, dass** die Membran eine Ausschluss-Obergrenze von 80kDa, vorzugsweise von 60kDa und besonders bevorzugt von 20kDa besitzt.

7. Probenahmevorrichtung nach einem der vorherigen Ansprüche, **dadurch gekennzeichnet, dass** die Membran aus einem Material besteht, das ausgewählt ist aus der Gruppe bestehend aus Cellulose und deren Derivate, Celluloseacetat, PTFE, Polycarbonat, Polypropylen, Polyamide und Polysulfone.

8. Probenahmevorrichtung nach einem der vorherigen Ansprüche, **dadurch gekennzeichnet, dass** die Membran mittels Klebeverfahren, durch Umspritzung mit dem Werkstoff, Vergießen oder durch lithografische Polymerisation des umliegenden Baukörpers fixiert wird.

9. Probenahmevorrichtung nach einem der vorherigen Ansprüche, **dadurch gekennzeichnet, dass** die Probenahmevorrichtung ein Probenahmekatheter, vorzugsweise für eine Blutuntersuchung und besonders bevorzugt ein zentralvenöser Katheter zur Anwendung an einem Säugetier, insbesondere einem Menschen, ist.

10. Probenahmevorrichtung nach einem der vorherigen Ansprüche, **dadurch gekennzeichnet, dass** die Zuleitung und die Ableitung in der Probenahmevorrichtung nebeneinander in einem einheitlichen Baukörper enthalten sind.

11. Probenahmevorrichtung nach einem der vorherigen Ansprüche, ferner umfassend einen Detektor zum Nachweisen des Analyten

12. Probenahmevorrichtung nach einem der vorherigen Ansprüche, **dadurch gekennzeichnet, dass** die Probenahmevorrichtung ferner eine Pumpe zum Pumpen eines Transportmediums durch die Zuleitung oder Ableitung versehen ist.

13. Probenahmevorrichtung nach Anspruch 12, **dadurch gekennzeichnet, dass** die Ableitung, optional über die Pumpe, in Fließverbindung mit dem Detektor steht.

14. Probenahmevorrichtung nach einem der Ansprüche 12 bis 13, ferner umfassend eine Steuerung zum Betätigen der Pumpe zu einem vorgewählten Zeitpunkt.

15. Probenahmevorrichtung nach Anspruch einem der Ansprüche 11 bis 14, **dadurch gekennzeichnet, dass** die Zu- und Ableitung über einen Verteiler mit dem Detektor derart verbunden sind, dass
- zum Spülen ein Transportmedium vom Verteiler ohne Passieren der Analytaufgabekammer zum Detektor geleitet werden kann, und
- zum Messen das Transportmedium vom Verteiler unter Passieren der Analytaufgabekammer zum Detektor geleitet werden kann.

16. Probenahmevorrichtung nach einem der vorherigen Ansprüche, ferner umfassend eine weitere Öffnung der Analytaufgabekammer, die mit einer analytdurchlässigen Membran versehen ist, und Mittel zum Beaufschlagen der weiteren Öffnung mit einem Medium zum Einstellen einer Analytkonzentration in der Analytaufgabekammer.

17. Verfahren zum Bestimmen eines Analyten in einem Medium, umfassend die Schritte:
a) Einführen einer Analytaufgabekammer einer Probenahmevorrichtung gemäß einem der Ansprüche 1 bis 16 in das zu analysierende Medium;
b) vor, während oder nach Schritt a) Befüllen der Analytaufgabekammer mit einem Transportmedium zur Aufnahme des Analyten;
c) Transportieren des Transportmediums aus der Analytaufgabekammer durch die Ableitung der Probenahmevorrichtung zu einem Detektor; und
d) Bestimmen des Analyten in dem abgeleiteten Transportmedium mit dem Detektor.
